# EUROPÄISCHE PATENTSCHRIFT

(11) **EP 2 522 274 B1**
(45) Veröffentlichungstag und Bekanntmachung des Hinweises auf die Patenterteilung: **17.06.2015**
(21) Anmeldenummer: 11006461.5
(22) Anmeldetag: 11.05.2011
(51) Int. Cl.: A43B 3/00, A43B 17/00, A61B 5/0488, A61N 1/36, A61H 39/00, A61B 5/103

(54) **System zur adaptiven Stimulation der Muskulatur**
System for adaptive stimulation of the muscular system
Système de stimulation adaptative de la musculature

(43) Veröffentlichungstag der Anmeldung: 14.11.2012
(62) Teilanmeldung aus: 11003858.5
(73) Patentinhaber: Haas, Helmut, 04552 Borna/OT Haubitz (DE); Görgner, Alexandra, 04229 Leipzig (DE)
(72) Erfinder: Haas, Helmut, 04552 Borna/OT Haubitz (DE); Görgner, Alexandra, 04229 Leipzig (DE)
(74) Vertreter: Dinter, Tilo

(56) Entgegenhaltungen:
- DE-A1- 10 143 268
- KR-A- 20090 089 528
- US-A1- 2006 282 017
- US-A1- 2008 033 505
- US-A1- 2009 005 834
- SCHIEPPATI M ET AL: "Early and late stretch responses of human foot muscles induced by perturbation of stance", EXPERIMENTAL BRAIN RESEARCH, SPRINGER INTERNATIONAL, DE, Bd. 105, Nr. 3, 1. Februar 1995 (1995-02-01), Seiten 411-422, XP008149664, ISSN: 0014-4819
- LOEB G E ET AL: "The functional reanimation of paralyzed limbs", IEEE ENGINEERING IN MEDICINE AND BIOLOGY MAGAZINE, IEEE SERVICE CENTER, PISACATAWAY, NJ, US, Bd. 24, Nr. 6, 1. September 2005 (2005-09-01), Seiten 45-51, XP001512614, ISSN: 0739-5175, DOI: 10.1109/MEMB.2005.1511499

## Beschreibung

Die Erfindung betrifft eine Einrichtung zur adaptiven Stimulation der Muskulatur, insbesondere zur Beeinflussung der Abrollparameter der Füße.

Bekannte Einrichtungen zur Stimulation der Muskulatur dienen vor allem zur Beeinflussung von Fehlhaltungen des Bewegungsapparates. So werden bei der Behandlung von Schlaganfall-Patienten mittels der Elektrostimulation zwecks Aktivierung der Muskeln die Reizsignale zum Gehirn geleitet. Die Patienten erhalten dadurch die Möglichkeit das Gehen neu zu erlernen. Dabei kommen im Stand der Technik kinematische Systeme zur Anwendung, bei denen zeitliche Druckverläufe des Fußes sowie Impulse und ungerichtete Kraftverläufe ermittelt und angezeigt werden. Mit diesen Parametern werden die Abrollverläufe der Füße beschrieben und mittels kinematischer Messverfahren ausgewertet. Ein zur Erfassung des Druckverlaufes der Füße dienendes Verfahren und eine dafür vorgesehene Vorrichtung zur Messwerterfassung und -auswertung in Schuhen bzw. Schuheinlagen wird nach der DE 102 01 134 A1 beschrieben. Bei diesem Verfahren erfolgt die Messdatenerfassung mittels mehrerer vorzugsweise piezoelektrischer Messfelder und der Verarbeitung der Messwerte in einer Auswerteeinheit. Mittels dieses Verfahrens können die aktuelle, die durchschnittliche Geschwindigkeit, die Anzahl der Schritte und die zurückgelegte Distanz als auch biomechanische Daten wie die Fußbelastung, der Abrollverlauf der Füße und eine eventuelle orthopädische Fehlbelastung oder Fehlstellung während eines Diagnoselaufes erfasst, analysiert, angezeigt und/oder gespeichert und einem Computer übermittelt werden. Bei der dabei zur Anwendung kommenden Vorrichtung erfolgt die Energieversorgung ohne den Einsatz von Batterien. Durch Nutzung von piezoelektrischen Effekten entstehen durch Erzeugung von Spannungssignalen unter Druckbelastung die Druckinformationen für die Sohlenbereiche. Gleichzeitig wird nach diesem Prinzip die Betriebsspannung für den Einchip-Computer erzeugt. Die Übertragung der Daten kann beispielsweise über Funksignale erfolgen. Nachteilig an dieser Lösung ist, dass sie ausschließlich auf die Messwerterfassung und -auswertung bezogen ist. Die anschließend erforderliche Elektrostimulation im klinischen Bereich macht die Behandlung aufwändig und ist nur im eingeschränkten Umfang möglich. Bekannt sind auch Stimulatoren für die Stimulation der Hautoberfläche. Bekannt sind weiterhin Neurostimulatoren sowie mit diesen in funktioneller Verbindung stehende Steuergeräte und Datenübertragungsverfahren wie sie nach der DE 101 43 268 A1 beschrieben werden. Diese Stimulatoren sind als Kanüle ausgebildet und sind zumindest teilweise in den Körper eines Patienten implantierbar. Eine elektronische Schaltung führt zur Stimulation den gegeneinander isolierten Kanülen unterschiedliche elektrische Signale zu. Eine mobile adaptive Stimulation der Muskelbereiche von Füßen ist mit dieser Einrichtung jedoch nicht möglich. Bei einer Einrichtung nach der US 2009/0005834 A1 beschriebenen Vorrichtung zur Vorbeugung von Thromboseerkrankungen werden durch Accelerometer Bewegungen der Beine erfasst und zur Muskelstimulierung der Beine Vibrationen erzeugt. Gesteuert wird die Vibrationsbehandlung durch einen Mikroprozessor. Für eine mobile Anwendung des Steuersystems zur adaptiven Steuerung der Stimulation der Muskelbereiche der Füße ist diese Lösung jedoch nicht geeignet. Nach der US 2006/0282017 A1 wird ein Kraftsensorsystem zur Anzeige der Druckverhältnisse an verschiedenen Bereichen des Körpers beschrieben. Mit diesem Sensorsystem wird die Kontrolle der Bewegungen durchgeführt. Eine Anwendung für adaptive Stimulierungen der Muskelbereiche der Füße ist nicht möglich. Ein Steuersystem zur elektromyographischen Messwerterfassung und Stimulation der Muskelbereiche von Füßen wird nach der KR 20090089528 beschrieben. Eine mobile adaptive Anpassung der Stimulationen durch eine dafür geeignete Vorrichtung ist jedoch nicht vorgesehen. Auch mit dem Muskel-Stimulationssystem nach der US 2008/0033505 A1 ist keine mobile adaptive Anwendung möglich. Diese Lösung beschreibt ein Kontrollsystem mit einer aufwendigen stationären Einrichtung, mit der die muskulären Reaktionen auf elektrische Stimulationen hin ermittelt und ausgewertet werden. Desweiteren wird nach der DE 20 2005 004 958 U1 eine Vorrichtung zur Stimulation der Hautoberfläche beschrieben, bei der Stimulations- Elemente in Form einer Matrix angeordnet sind und jedes Element an einem vorgegebenen Bereich der Hautoberfläche ein Stimulussignal anlegt. Die Stimulations-Elemente sind mit einer Steuerungseinheit (Controller) verbunden, der diese so steuert, dass jede Art von Stimulationssignalen angelegt werden kann. So können durch eine entsprechende Ansteuerung verschiedene Muster von Stimulationssignalen angelegt werden. Die Muster können dabei eine Vielzahl von Stimulussignalen aufweisen, die durch eine parallele, simultane, sequenzielle oder kombinatorisch miteinander verbundene Steuerungsart erzeugt werden. Bei der Anwendung der Stimulationsvorrichtung in Form einer "Sohle" zur Fußstimulation wird das Muster der zu steuernden Stimulations-Elemente für die vorgegebenen Fußbereiche vom Controller vorgegeben. Obwohl universelle Steuerungsmöglichkeiten durch diese Steuerungsvorrichtung gegeben sind, so ist auch die Anwendung dieser Lösung durch die erforderliche stationäre Behandlung aufwändig und mit Einschränkungen verbunden. Bekannt sind auch Vorrichtungen zur Behandlung biologischen Gewebes mittels magnetischer Felder. Eine derartige Vorrichtung zur Magnetstimulation wird beispielsweise nach der WO 2011/045002 beschrieben. Sie dient für prophylaktische und therapeutische Anwendungen im physiotherapeutischen, orthopädischen sowie dermatologischen Bereich. Mit dieser Vorrichtung sollen die bei der konventionellen Elektrostimulation erforderlichen hohen Stromstärken bzw. Spannungen vermieden und das Einsatzgebiet über den peripheren Bereich des zu behandelnden Organismus hinaus erweitert werden. Die Anwendung der Vorrichtung soll auch für die Stimulation von tief unter der Haut liegenden Nerven- und Muskelzellen möglich sein. Nachteilig ist jedoch an dieser Vorrichtung die Beschränkung der Anwendung und der damit verbundenen Wirkungen ausschließlich auf den Einsatz magnetischer Felder. Weiterhin bekannt ist nach der US 6,273,863 B1 ein tragbares selbstlernendes adaptives Überwachungssystem zur Anwendung bei der Rehabilitation orthopädischer Patienten mit Frakturen der unteren Extremitäten. Bei diesem System kommt eine flexible Einlegesohle zur Anwendung. Die Einlegesohle weist Druck- und/oder Kraftsensoren auf, mit denen die Druckkräfte und die Bodenreaktionskräfte während des Bewegungsvorganges an den Füßen erfasst werden. Wird vom Patienten die vorgegebene physiologische Belastung überschritten, so wird ein elektrisches Signal erzeugt, dieses digitalisiert, gemittelt und verstärkt sowie danach grafisch oder akustisch aufbereitet. Dem Patienten wird so die ausgeübte Kraft im Form eines sogenannten "biofeedback" mitgeteilt. Dadurch wird vermieden, dass der Patient mit einer Knochenfraktur oder einer Amputation die Wundnaht oder den Knochen zu früh oder zu stark belastet. Auftretende Komplikationen, Fehlstellungen und Hautdefekte können so vermieden werden. Auch bei diesem System werden hauptsächlich belastete Fußbereiche, die sich im vorderen Quergewölbe und im Fersenbereich des Fußes befinden, stimuliert. Nach der erfolgten Signalisierung für den Patienten die Leistung umzuverteilen, muss er in der Lage sein, die Belastung selbstständig zu regulieren. Dies erweist sich für die Mehrheit der Patienten, wie beispielsweise Schlaganfallpatienten, als nicht anwendbar. Die Aufgabe der Erfindung besteht deshalb in der Schaffung einer Einrichtung zur adaptiven Stimulation der Muskulatur, die mobil, also während des Bewegungsvorganges, angewendet werden kann und dabei unabhängig von der Änderungsmöglichkeit der Belastung durch den Patienten anwendbar ist. Gelöst wird diese Aufgabe mittels der geschaffenen Einrichtung mit den beschreibenden Merkmalen nach Patentanspruch 1. Vorteilhafte Weiterbildungen der Einrichtung werden durch die Merkmale der Patentansprüche 2 bis 5 gekennzeichnet. In der erfindungsgemäßen Einrichtung sind in Schuheinlagen Baugruppen eingebracht, die zur elektromyographischen Messwertaufnahme dienen. Dabei wird beim Gehen des Patienten neben der Erfassung des Druckverlaufes auch die Elektrostimulation an vorgegebenen Stellen des Fußbereiches durchgeführt. Dazu werden die von den in der Schuheinlage angeordneten Drucksensoren erfassten Messwerte zur Elektrosimulation genutzt. Während des Gehens erhalten die vorgegebene Bereiche des Fußes eine permanente Stimulation durch die der Patient das Gehen neu erlernen kann. Zu diesem Zweck ist in der Auswertebaugruppe (Controller) ein vorher ermitteltes individuelles Stimulationsmuster abgespeichert. Mittels dieses Stimulationsmusters werden separate Stimulatoren zur Stimulation in unterschiedliche Stärkegraden durch die Auswertebaugruppe angeregt. Um hierbei auch Ermüdungserscheinungen und andere Randbedingungen zu berücksichtigen, wird ein Abfall der Stimulationsfrequenz über die Zeit einbezogen. Durch einen bevorzugt zyklischen Vergleich zwischen dem vorgegebenen abgespeicherten Muster physiologischer Muskelaktivitäten mit den gemessenen elektromyographischen Werten werden optimale Stimulationssignale erzeugt. Die jeweiligen zu vorgegebenen Zeitpunkten gemessenen elektromyographischen Werte werden in einer mit der Ausgabebaugruppe verbundenen Speicherbaugruppe abrufbar abgespeichert. Vorteilhaft können diese Daten mittels einer RFID-Baugruppe übertragen und ausgewertet werden. Sie dienen zur Bewertung des Abrollverhaltens des Fußes und zur Verfeinerung der Kalibrierung des Systems.

Eine beispielsweise Form der Einrichtung besteht in der kombinierten Ausbildung der Messwertaufnehmer und der Stimulationselemente als eine Baugruppe. Zur Anwendung kommen dabei matrixförmig angeordnete piezoelektrische Baugruppen, die sich im jeweiligen inneren Bereich der kombinierten Baugruppe befinden und die Stimulationselemente ringförmig um die piezoelektrischen Baugruppen angeordnet sind. Die jeweiligen piezoelektrischen Baugruppen sind über die Auswertebaugruppe (Controller) direkt mit dem jeweiligen zugeordneten Stimulationselement elektrisch verbunden. Messwertänderungen der Drucksensoren haben so unter Einbeziehung der vorgegebenen Stimulationsmuster durch die Auswertebaugruppe (Controller) einen unmittelbaren Einfluss auf die Steuerung der Hautstimulationen.

## Patentansprüche

1. Einrichtung zur adaptiven Stimulation der Muskulatur mit einer in Schuheinlagen angeordneten Baugruppe zur elektromyographischen Messwertaufnahme, einer mit einer Speicherbaugruppe verbunden Auswertebaugruppe bzw. Controller und einer zur Muskelstimulation dienenden Baugruppe, wobei die Auswertebaugruppe bzw. der Controller dafür ausgelegt ist, die Differenzwerte zwischen den elektromyographischen Messwerten und den Sollwerten eines vorgegebenen Sollwertprofiles zu ermitteln und daraufhin die Stimulation bestimmter Muskelbereiche der Füße mittels der zur Muskelstimulation dienenden Baugruppe zu steuern, sowie die Auswertebaugruppe bzw. der Controller zur abrufbaren Speicherung der zyklisch erfassten elektromyographischen Werte mit der Speicherbaugruppe verbunden ist.

2. Einrichtung nach Patentanspruch 1, **dadurch gekennzeichnet, dass** der Speicherbaugruppe eine zur Übertragung der abgespeicherten Daten dienende RFID-Baugruppe zugeordnet ist.

3. Einrichtung nach Patentanspruch 1 , **dadurch gekennzeichnet, dass** die zur elektromyographischen Messwertaufnahme dienende Baugruppe und die zur Muskelstimulation dienende Baugruppe durch eine Baugruppe gebildet werden, deren Funktionsänderungen durch Änderung der Ansteuersignale vornehmbar ist.

4. Einrichtung nach Patentanspruch 1, **dadurch gekennzeichnet, dass** die zur elektromyograhischen Messwertaufnahme dienende Baugruppe aus matrixförmig angeordneten Messwertaufnehmern besteht.

5. Einrichtung nach Patentanspruch 1, **dadurch gekennzeichnet, dass** die zur Muskelstimulation dienende Baugruppe aus matrixförmig angeordneten Stimulatoren besteht

## Claims

1. Device for adaptive muscle stimulation that includes a component for the electromyographic recording of measured values arranged in shoe inserts, an evaluation module and/or controller with storage component and a component for muscle stimulation, whereas the evaluation module and/or controller is designed to define the differences between the electromyographic values and the nominal values stipulated by a predefined nominal value profile and to then control the stimulation of specific foot muscle areas through the muscle stimulating component as well as the evaluation module and/or controller for the retrievable storage of cyclically recorded electromyographic values linked to the storage component.

2. Device as per patent claim 1, **characterized by** the fact that an RFID component serving to transmit the data stored is associated with the storage component.

3. Device as per patent claim 1, **characterized by** the fact that the component for the electromyographic recording of measured values and the component for muscle stimulation are made up of a component the functions of which can be changed by changing the control signals.

4. Device as per patent claim 1, **characterized by** the fact that the component for the electromyographic recording of measured values is composed of readings recorders in matrix-shaped design.

5. Device as per patent claim 1, **characterized by** the fact that the component for muscle stimulation is composed of stimulators in matrix-shaped design.

## Revendications

1. Dispositif de stimulation musculaire adaptive avec module de mesure électromyographique disposé dans une semelle de chaussure et qui relie un module d'enregistrement et un module d'analyse, soit, un controlleur ainsi qu'un module prévu pour la stimulation; en même temps que le module analytique ou controlleur sont prévus pour communiquer les valeurs différencielles des valeurs de mesures électromyographiques ainsi que les valeurs de points de consigne d'un profil donné de valeurs de point de consigne, et, de cette manière, peuvent contrôler le module servant à la stimulation de zones musculaires précises des pieds et également relier les groupes analytiques au controlleur pour l'enregistrement et la disposition des valeurs recueillies de manière cyclique à l'aide du module.

2. Dispositif d'après la revendication 1, **caractérisé en ce que**, le module d'enregistrement soit assigné à un module RFID servant à communiquer les données enregistrées.

3. Dispositif d'après la revendication 1, **caractérisé en ce que**, le module servant à l'enregistrement des valeurs de mesures électromyographiques ainsi que le module servant à la stimulation musculaire, forment un ensemble modulaire dont les changements de fonction puissent être effectués en modifiant les signaux de commande.

4. Dispositif d'après la revendication 1, **caractérisé en ce que**, le module voué à l'enregistrement des valeurs de mesures électromyographiques soit composé de capteurs disposés sous forme de matrice.

5. Dispositif d'après la revendication 1, **caractérisé en ce que**, le module visant à la stimulation musculaire soit composé de stimulateurs disposés sous forme de matrice.
